# EUROPÄISCHE PATENTSCHRIFT

(11) **EP 1 297 816 B1**
(45) Veröffentlichungstag und Bekanntmachung des Hinweises auf die Patenterteilung: **25.02.2004**
(21) Anmeldenummer: 02018430.5
(22) Anmeldetag: 16.08.2002
(51) Int. Cl.: A61K 7/06

(54) **Perlglänzende Haarkur**
Pearlescent hair treatment composition
Composition de traitement des cheveux nacrée

(30) Priorität: 26.09.2001 DE 10147501
(43) Veröffentlichungstag der Anmeldung: 02.04.2003
(73) Patentinhaber: Wella Aktiengesellschaft, 64274 Darmstadt (DE)
(72) Erfinder: Pfaffernoschke, Matthias, Dr., 3270 Aarberg (CH); Jungo, Sybille, 1717 St. Ursen (CH)

(56) Entgegenhaltungen:
- EP-A- 0 376 083
- EP-A- 0 407 042
- WO-A-01/28505
- WO-A-01/52800
- WO-A-96/40815

## Beschreibung

Gegenstand der Erfindung ist ein Haarbehandlungsmittel, welches insbesondere als Leave-in Haarkur oder als Haarspülung anwendbar ist, in Form eines perlgänzenden Produktes vorliegt und einen Gehalt an bestimmten Assoziativverdickern, kationischen Haarpflegewirkstoffen sowie bestimmten Perlglanz- oder Trübungsmitteln aufweist.

Übliche haarkonditionierende Präparate wie Rinse-off Kuren oder Leave-on Treatments sind in der Regel auf der Basis von wässrigen Emulsionen formuliert. Wesentliche Inhaltsstoffe sind kationaktive Substanzen, insbesondere kationische Tenside, hydrophobe Substanzen, insbesondere Fettalkohole, Emulgatoren, sowie weitere spezifische Wirk- und Duftstoffe. Die wichtigsten Bestandteile sind dabei kationische Tenside, Fettalkohole und Emulgatoren. Die Präparate sind von milchig-weißem Aussehen und können zur Verbesserung des Perlglanzeffektes Perlglanzmittel enthalten. Einen Überblick über den prinzipiellen Aufbau von Kurspülungen und Haarkuren gibt Schrader, 'Grundlagen und Rezepturen der Kosmetika', 2. Auflage, 1989, Seiten 728 bis 737. Hauptaufgaben der Konditioniermittel sind die Verbesserung der Frisierbarkeit, der Kämmbarkeit, des Glanzes und des Griffs des behandelten Haares.

Die bekannten emulsionsförmigen Haarkuren auf Basis von Kationtensiden und Fettalkoholen weisen produktionstechnische Probleme aufweisen. Die in der Regel festen Fettalkohole müssen aufgeschmolzen und bei höheren Temperaturen ein- emulgiert werden. Der Herstell- und Abkühlvorgang hat einen erheblichen Einfluß auf die Produktqualität, insbesondere auf die Produktkonsistenz und auf die Qualität des Perlglanzeffektes. Der Perlglanz ist stark vom Temperaturverlauf während der Abkühlung abhängig. In der Abkühlphase wird ein viskoelastischer Zustand durchlaufen und es kann zu Problemen der Auskristallisation bei höheren Temperaturen kommen. Der Temperaturverlauf während der Abkühlung ist aber häufig unvorhersehbar und schwer reproduzierbar zu steuern. Dies hat zur Folge, dass Emulsionsstabilität, Viskosität und Perlglanzeffekt erheblichen Schwankungen von einem Produktionsansatz zu einem anderen aufweisen und bei manchen Chargen unzureichend sind. Eine nachträgliche Verdickung ist in der Regel nicht möglich.

Es bestand deshalb die Aufgabe, ein haarkonditionierendes Haarpflegemittel zu entwickeln, welches einerseits einen schönen Perlglanzeffekt aufweist, mindestens gleich gute haarkonditionierende Eigenschaften aufweist wie eine herkömmliche Haarkur auf Basis einer wässrigen Emulsion von Kationtensid und Fettalkohol und gleichzeitig eine bessere Stabilität hinsichtlich Perlglanz und Viskosität aufweist und einfacher mit zuverlässigerer Qualität herstellbar ist.

In der WO 01/28505 A1 werden klare Haarbehandlungsmittel beschrieben mit einem Gehalt an u.a. einem nichtionischen, amphiphilen Assoziativverdicker, ausgewählt aus hydrophob modifizierten Aminoplast/Polyether Copolymeren. Die Mittel können zusätzlich kationische Gruppen aufweisende haarpflegende Stoffe enthalten, z.B. kationische Tenside, kationische Polymere, kationisch derivatisierte Proteine, kationisch derivatisierte Proteinhydrolyste oder Betain. In der EP 0 376 083 A2 werden fliessfähige Perlglanzkonzentrate beschrieben. Die Perlglanzkonzentrate eignen sich zur Herstellung getrübter oder perlglänzender flüssiger, wässriger Zubereitungen wie z.B. Haarspülmitteln.

Es wurde nun gefunden, dass die Aufgabe gelöst wird durch ein Haarbehandlungsmittel der nachfolgend beschriebenen Zusammensetzung. Gegenstand der Erfindung ist ein perlglänzendes Haarpflegemittel mit einem Gehalt an
(A) mindestens einem nichtionischen, amphiphilen Assoziativverdicker, ausgewählt aus hydrophob modifizierten Aminoplast/Polyether Copolymeren,
(B) mindestens einem Haarpflegewirkstoff, ausgewählt aus kationischen Tensiden, zwitterionischen Tensiden, kationischen Polymeren, kationischen Silikonverbindungen, aminsubstituierten Silikonverbindungen, kationisch derivatisierten Proteinen, kationisch derivatisierten Proteinhydrolysaten und Betain und
(C) mindestens einem Perlglanz- oder Trübungsmittel, ausgewählt aus Fettsäurealkanolamiden, Fettsäureglycerylestern, Guanin, Glykoldifettsäureestern, Styrol/Acrylat Copolymeren, Polyethylenglykoldifettsäureestern, Styrol/Vinylpyrrolidon Copolymeren und Poly(trimethylammoniumethylmethacrylat Chlorid)
in einer wässrigen, kosmetischen Basis.

Der Assoziativverdicker (A) ist in dem erfindungsgemäßen Mittel in einer Menge von vorzugsweise 0,1 bis 5 Gew.%, besonders bevorzugt von 0,1 bis 2 Gew.% enthalten. Der Haarpflegewirkstoff (B) ist vorzugsweise in einer Menge von 0,01 bis 10, besonders bevorzugt von 0,1 bis 5 Gew.% enthalten. Das Perlglanz- oder Trübungsmittel (C) ist in einer Menge von vorzugsweise 0,1 bis 10 Gew.%, besonders bevorzugt von 0,5 bis 6 Gew.% enthalten. Dass Mittel ist vorzugsweise im wesentlichen frei von Fettalkoholen, d.h. es enthält keine oder weniger als 0,2 Gew.% Fettalkohole.

Das Mittel erfüllt die an ein Haarkonditioniermittel hinsichtlich Konditionierwirkung zu stellenden Anforderungen in bester Weise, weist einen sehr guten Perlglanzeffekt auf und zeichnet sich durch eine zuverlässige Herstellbarkeit und eine verbesserte Produktstabilität aus. Auch bei längerer Lagerung bei erhöhten Temperaturen ist das erfindungsgemäße Mittel hinsichtlich Produktkonsistenz und Perglanz stabil. Mit dem erfindungsgemäßen Mittel behandeltes Haar ist sowohl im feuchten als auch im trockenen Zustand merkbar glatter und die Nasskämmbarkeit ist merkbar verbessert. Überraschenderweise wurde gefunden, dass diese Effekte auch ohne die in herkömmlichen Kuren unverzichtbaren Fettalkohole erzielt werden. Der über längere Zeit stabile und besonders schöne Perlglanzeffekt ermöglicht es, das erfindungsgemäße Mittel in eine optisch ansprechende, durchsichtige oder durchscheinende Verkaufsverpackung aus z.B. Glas oder einem durchsichtigem Kunststoff wie z.B. Polyethylen, Polypropylen oder Polyethylenterephtalat abzufüllen.

Der nichtionische, amphiphile Assoziativverdicker (A) ist ein Polymer, welches sowohl hydrophile als auch hydrophobe Gruppen enthält. Assoziativverdicker sind wasserlösliche Polymere und haben tensidartige hydrophobe Bestandteile, welche in der Lage sind, sich in einem hydrophilen, insbesondere wäßrigen Medium sowohl mit sich selbst als auch mit anderen hydrophoben Stoffen zu assoziieren, d.h. in Wechselwirkung zu treten. Durch das daraus resultierende assoziative Netzwerk wird das Medium verdickt oder geliert. Typischerweise werden Assoziativverdicker hergestellt durch Polymerisation von Polyethylenoxid-Prepolymeren und mindestens zweifach funktionellen, polykondensierbaren Stoffen wie z.B. Isocyanaten, wobei Mono- oder Diole mit großen Aryl-, Alkyl- oder Aryl/Alkyl-Gruppen eingebaut werden, um die hydrophobe Modifikation bereitzustellen. Bevorzugte Assoziativverdicker sind daher hydrophob modifizierte Polyalkylenglykole. Hierbei wird der hydrophile Bestandteil durch Polyoxyalkyleneinheiten, vorzugwseise Polyoxyethylen- aber auch Polyoxypropyleneinheiten oder deren Gemisch gebildet. Der hydrophobe Bestandteil wird vorzugsweise aus Kohlenwasserstoffgruppen, z.B langkettigen Alkyl-, Alkylaryl- oder Arylalkylgruppen gebildet. Geeignete Assoziativverdicker sind Reaktionsprodukte der säurekatalysierten Reaktion von Glycolurilderivaten und Polyalkylenglykolen und alkoxylierten Kohlenwasserstoffen.

Die Assoziativverdicker sind hydrophob modifizierte Aminoplast-Polyether Copolymere. Bezüglich deren Struktur und Herstellung wird auf die WO 96/40815 verwiesen. In der WO 96/40815 werden wasserdispergierbare oder wasserlösliche Copolymere beschrieben, welche die Reaktionsprodukte sind einer säurekatalysierten Polykondensation von mindestens zweifach funktionellen Aminoplastmonomeren und mindestens zweifach funktionellen Alkylenpolyethern sowie einfach funktionellen Verbindungen mit hydrophoben Gruppen. Geeignete Aminoplaste sind der Figur 1 der WO 96/40815 zu entnehmen:

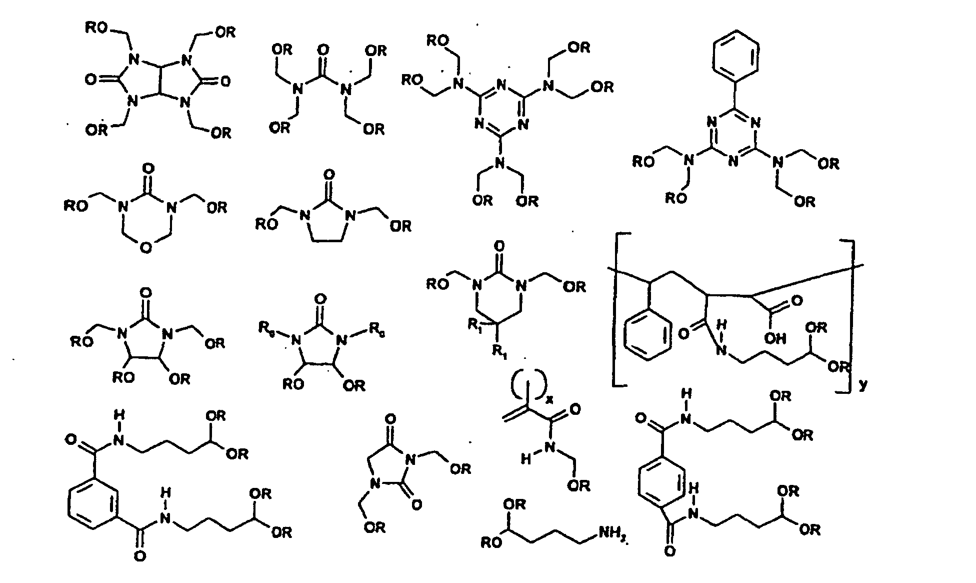

Wobei R Wasserstoff, Alkyl mit 1 bis 4 C-Atomen oder Acyl mit 1 bis 4 C-Atomen ist; R₀ Alkyl mit 1 bis 4 C-Atomen, Aryl, Cycloalkyl ist; R₁ Alkyl mit 1 bis 4 C-Atomen ist; und x 0 oder 1 ist und y mindestens 2 ist.

Besonders bevorzugt sind die Glycolurilderivate der Formel X der WO 96/40815: 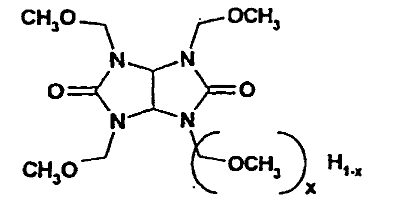 wobei x die gleiche Bedeutung hat wie oben.

Geeignete Alkylenpolyether sind der Figur 2 der WO 96/40815 zu entnehmen: 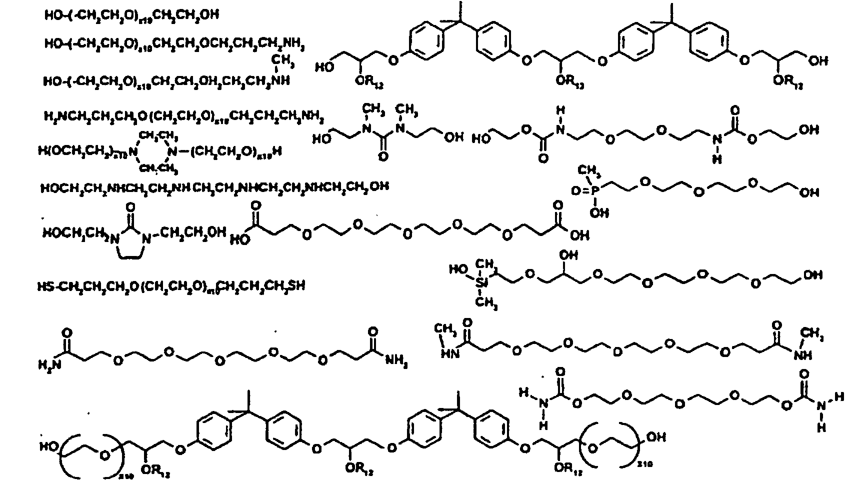 wobei x10 einen Wert von ungefähr 1 bis ungefähr 100 hat und R₁₂ Alkyl mit 1 bis 4 C-Atomen oder Acyl mit 1 bis 3 C-Atomen ist.

Bevorzugte Alkylenpolyether sind Polyethylenoxiddiole. Diese können einen Ethoxylierungsgrad von 20 bis 500, vorzugsweise 50 bis 350, besonders bevorzugt von 100 bis 250 haben. Geeignete einfach funktionelle Verbindungen mit hydrophoben Gruppen sind diejenigen der Formel XIV der WO 96/40815:

(R₀₉)ₓ₁₃ - (R₁₀)ₓ₁₄- (R₁₁)ₓ₁₅- (R₁₂)ₓ₁₆-Y

wobei R₀₉ Wasserstoff, Alkyl mit 8 bis 24 C-Atomen, Alkenyl mit 8 bis 24 C-Atomen oder Alkinyl mit 8 bis 24 C-Atomen ist; R₁₀ Mono-, Di- oder Tri (aryl) ist; R₁₁ Aryl, Mono-, Di- oder Tri(alkaryl), Mono-, Di- oder Tri(alkcycloalkyl), Alkenyl oder Alkinyl ist, wobei Alkyl, Alkenyl und Alkinyl 1 bis 24 C-Atome aufweisen und Cycloalkyl 4 bis 8 C-Atome aufweist; R₁₂ ist ein oder mehr Alkylenoxid; Y ist eine ein aktives Wasserstofftaom aufweisende Gruppe wie OH, SH, COOH, CONHR₀₈; x13, x14, X15 und X16 sind 0 oder 1, wobei zwei oder mehr von x13, x14, X15 und X16 gleichzeitig 1 sind.

Erfindungsgemäß geeignete Assoziativverdicker sind vorzugsweise ausgewählt aus Polymeren der allgemeinen Formel (I) 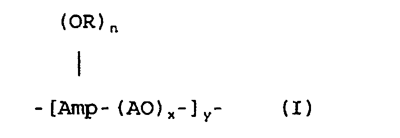 wobei Amp ein Aminoplastmonomer oder den Rest eines Aminoplastoligomers oder -polymers bedeutet,
AO für eine Alkylenoxidgruppe steht, R für Wasserstoff, C1-C4-Alkyl oder C1-C4-Acyl steht, x und y Zahlen größer 1 sind und n eine positive Zahl ist.

Besonders bevorzugt sind die Reaktionsprodukte der säurekatalysierten Polykondensation von (a) Glykolurilen der Formel (II) 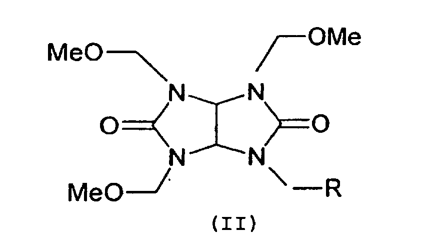 wobei R für H oder vorzugsweise für OMe steht mit (b) Polyethylenoxiddiolen eines Ethoxylierungsgrades von 20 bis 500, vorzugsweise 50 bis 350, besonders bevorzugt von 100 bis 250 sowie (c) eines gegebenenfalls ethoxylierten hydrophoben Alkohols, Alkylphenols, Thiols, Carboxamids, Carbamats oder einer hydrophoben Carbonsäure, wie sie auf den Seiten 17 bis 19 der WO 96/40815 beschrieben sind: 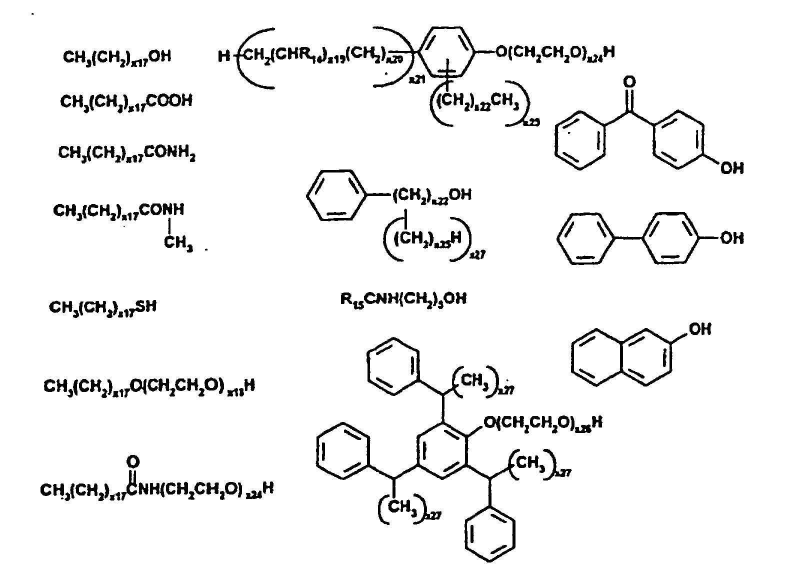 wobei R₁₄ Wasserstoff, oder Alkyl mit 1 bis 12 C-Atomen ist; R₁₅ Aryl oder Alkyl mit 8 bis 24 C-Atomen ist; X17 einen Wert von 7 bis 23 hat; x18 einen wert von 1 bis ungefähr 20 hat; x19 einen Wert von 0 bis ungefähr 8 hat; x20 0 oder 1 ist; x21 0 oder 1 ist; x22 einen Wert von 1 bis ungefähr 20 hat; x23 einen Wert von 1 bis ungefähr 23 hat; x24 einen Wert von 1 bis ungefähr 120 hat; x25 einen Wert von 1 bis ungefähr 20 hat; x26 einen Wert von ungefähr 8 bis ungefähr 60 hat und x 27 0 oder 1 ist; die Summe von x19 und x20 1 bis ungefähr 23 ist; und die Summe von X22 und x25 1 bis ungefähr 20 ist. Eine weitere Gruppe hydrophober Stoffe basiert auf teilverseiften Fettsäureglyceriden, wie z.B. teilverseiftem Leinsamenöl, Tallöl, Baumwollsamenöl, Rizinusöl, Kokosöl, Maisöl, Oiticicaöl, Perilaaöl, Mohnsamenöl, Rapsöl und ähnlichen. Eine weitere Gruppe hydrophober Stoffe sind Ethoxylate der teilverseiften Fettsäureglyceride. Beispielhaft für solche Ester sind

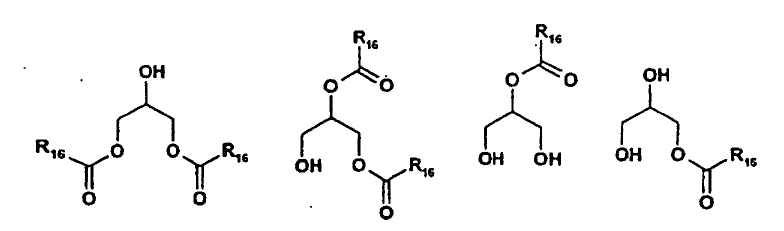

Wobei R₁₆ der Hydrocarbylteil der natürlichen Fettsäurekomponente der Fettsäureglyceride ist. Beispielhaft für deren Ethoxylate sind

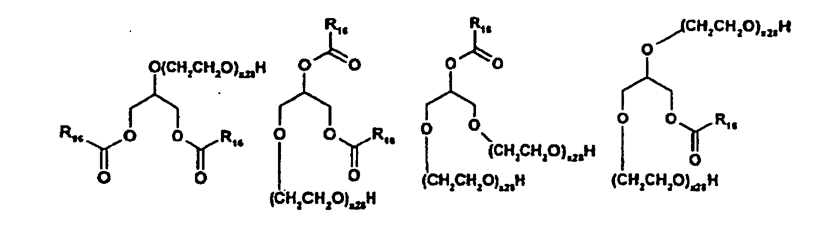

Wobei x28 einen Wert von 1 bis ungefähr 200 hat und R16 die natürliche Fettsäurekomponente des natürlichen Öls ist.

Besonders bevorzugtes Glykoluril ist 1,3,4,6-Tetramethoxymethylglycoluril.

Geeignete Assoziativverdicker sind solche mit den INCI-Bezeichnungen Polyether-1, PEG-180/Octoxynol-40/TMMG Copolymer und PEG-180/Laureth-50/TMMG Copolymer und werden von der Firma Süd-Chemie vertrieben unter den Handelsbezeichnungen Pure-Thix® HH, L und M.

Der Haarpflegewirkstoff (B) ist eine Substanz, die auf Grund von kationischen oder kationisierbaren Gruppen, insbesondere primären, sekundären, tertiären oder quaternären Amingruppen eine Substantivität zu menschlichem Haar aufweist. Geeignete kationaktive Stoffe sind ausgewählt aus kationischen Tensiden, zwitterionischen, insbesondere betainischen Tensiden, kationischen Silikonverbindungen, aminsubstituierten Silikonverbindungen, kationaktiven organischen Polymeren mit kationischen oder kationisierbaren Gruppen, kationisch derivatisierten Proteinen, kationisch derivatisierten Proteinhydrolysaten und Betain.

Geeignete kationische Tenside sind Tenside, welche eine quaternäre Ammoniumgruppe enthalten. Bevorzugte kationische Tenside sind solche der allgemeinen Formel (III).

N⁽⁺⁾R¹R²R³R⁴ X⁽⁻⁾ (III)

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X ⁽⁻⁾ ein kosmetisch verträgliches Anion darstellt, beispielsweise ein Halogen, Acetat, Phosphat, Nitrat oder Alkylsulfat, vorzugsweise ein Chlorid. Mindestens einer der Substituenten R1 bis R4 ist eine Alkyl- oder eine einfach oder mehrfach ungesättigte Alkenylgruppe mit mindestens 8 C-Atomen. Bevorzugt sind Fettalkyltrimethylammonium- und Difettalkyldimethylammoniumverbindungen. Beispiele für geeignete kationische Tenside sind die Chloride oder Bromide von Alkyldimethylbenzylammoniumsalzen, Alkyltrimethylammoniumsalze, z.B. Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, z.B. Lauryl- oder Cetylpyridiniumchlorid, Alkylamidoethyltrimethylammoniumethersulfate sowie Verbindungen mit kationischem Charakter wie Aminoxide, z.B. Alkylmethylaminoxide oder Alkylaminoethyldimethylaminoxide. Besonders bevorzugt sind Cetyltrimethylammoniumchlorid, Behenyltrimethylammoniumchlorid und Stearyltrimethylammoniumchlorid.

Geeignete zwitterionische Tenside sind Derivate aliphatischer quaternärer Ammonium-, Phosphonium- und Sulfoniumverbindungen der Formel (IV) 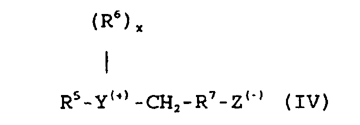 wobei R5 eine geradkettige oder verzweigtkettige Alkyl-, Alkenyl- oder Hydroxyalkylgruppe mit 8 bis 18 C-Atomen und 0 bis etwa 10 Ethylenoxideinheiten und 0 bis 1 Glycerineinheiten darstellt; Y eine N-, P- oder S-haltige Gruppe ist; R6 eine Alkyl- oder Monohydroxyalkylgruppe mit 1 bis 3 C-Atomen ist; x gleich 1 ist, falls Y ein Schwefelatom ist und x gleich 2 ist, wenn Y ein Stickstoffatom oder ein Phosphoratom ist; R7 eine Alkylen- oder Hydroxyalkylengruppe mit 1 bis 4 C-Atomen ist und Z⁽⁻⁾ eine Carboxylat-, Sulfat-, Phosphonatoder Phosphatgruppe darstellt. Andere amphotere Tenside wie Betaine sind ebenso geeignet für das erfindungsgemäße Haarbehandlungsmittel. Beispiele für Betaine umfassen C8- bis C18-Alkylbetaine wie Cocodimethylcarboxymethylbetain, Lauryldimethylcarboxymethylbetain, Lauryldimethylalphacarboxyethylbetain, Cetyldimethylcarboxymethylbetain, Oleyldimethylgammacarboxypropylbetain und Lauryl-bis(2-hydroxypropyl)-alphacarboxyethylbetain; C8- bis C18-Sulfobetaine wie Cocodimethylsulfopropylbetain, Stearyldimethylsulfopropylbetain, Lauryldimethylsulfoethylbetain, Laurylbis-(2-hydroxyethyl)-sulfopropylbetain; die Carboxylderivate des Imidazols, die C8- bis C18-Alkyldimethylammoniumacetate, die C8- bis C18-Alkyldimethylcarbonylmethylammoniumsalze sowie die C8- bis C18-Fettsäurealkylamidobetaine wie z.B. das Kokosfettsäureamidopropylbetain, welches beispielsweise in Form einer 30%igen wäßrigen Lösung unter der Handelsbezeichnung Tego® Betain L7 von der Firma Goldschmidt AG vertrieben wird und das N-Kokosfettsäureamidoethyl-N-[2-(carboxymethoxy)ethyl]-glycerin (CTFA-Name: Cocoamphocarboxyglycinate), welches z.B. in Form einer 50%igen wäßrigen Lösung unter der Handelsbezeichnung Miranol® C2M von der Firma Miranol Chemical Co. Inc. vertrieben wird. Besonders bevorzugt ist Kokosfettsäureamidopropylbetain.

Bei den geeigneten kationischen Polymeren handelt es sich vorzugsweise um haarfestigende oder um haarkonditionierende Polymere. Geeignete Polymere der Komponente (B) enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind z.B. die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11). Von den kationischen Polymeren, die in dem erfindungsgemäßen Mittel enhalten sein können, ist z.B. Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und von denen das Gafquat® 734 besonders bevorzugt ist, geeignet. Weitere kationische Polymere sind z.B. das von der Firma BASF, Deutschland unter dem Handelsnamen LUVIQUAT® HM 550 vertriebene Copolymer aus Polyvinylpyrrolidon und Imidazoliminmethochlorid, das von der Firma Calgon/USA unter dem Handelsnamen Merquat® Plus 3300 vertriebene Terpolymer aus Dimethyldiallylammoniumchlorid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam und das von der Firma Gaf unter dem Handelsnamen Gafquat® HS 100 vertriebene Vinylpyrrolidon/Methacrylamidopropyltrimethylammoniumchlorid Copolymer.

Geeignete kationische Polymere, die von natürlichen Polymeren abgeleitet sind, sind kationische Derivate von Polysacchariden, z.B. kationische Derivate von Cellulose, Stärke oder Guar. Geeignet sind weiterhin Chitosan und Chitosanderivate. Kationische Polysaccharide haben die allgemeine Formel (V)

G- (O-B-N⁺R^{a}R^{b}R^{c})ₙ X⁻ (V)

G ist ein Anhydroglucoserest, z.B. Stärke- oder Celluloseanhydroglucose und n ist eine Zahl größer Null;
B ist eine divalente Verbindungsgruppe, z.B. Alkylen, Oxyalkylen, Polyoxyalkylen oder Hydroxyalkylen;
R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X⁽⁻⁾ ist ein übliches Gegenanion, hat die gleiche Bedeutung wie bei Formel (III) und ist vorzugsweise Chlorid. Eine kationische Cellulose wird unter der Bezeichnung Polymer JR von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar® R vertrieben und hat die INCI-Bezeichnung Guar Hydroxypropyltrimonium Chloride.

Besonders bevorzugte kationaktive Stoffe sind neutralisierte Chitosane und Chitosan-Derivate. Hierbei handelt es sich um vollständig oder partiell deacetylierte Chitine. Das Molekulargewicht des Chitosans kann über ein breites Spektrum verteilt sein, beispielsweise von 20.000 bis ca. 5 Millionen g/mol. Geeignet ist z.B. ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%. Ein geeignetes Chitosan wird z.B. von der Firma Kyowa Oil&Fat, Japan, unter dem Handelsnamen Flonac® vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches z.B. unter der Bezeichnung Kytamer® PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quaternierte, alkylierte oder hydroxyalkylierte Chitosane, z.B. Hydroxyethyl- oder Hydroxybutylchitosan in Betracht. Die Chitosane oder Chitosanderivate liegen in neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie z.B. Ameisensäure, Weinsäure, Äpfelsäure, Milchsäure, Zitronensäure, Pyrrolidoncarbonsäure, Salzsäure u.a., von denen Pyrrolidoncarbonsäure und Milchsäure besonders bevorzugt sind.

Weitere geeignete kationische Haarpflegewirkstoffe sind kationisch modifizierte Proteinderivate oder kationisch modifizierte Proteinhydrolysate und sind beispielsweise bekannt unter den INCI-Bezeichnungen Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein oder Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, Hydroxypropyltrimonium Hydrolyzed Vegetable Protein.

Geeignete kationisch derivatisierte Proteinhydrolysate sind Substanzmischungen, die z.B. durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können. Proteine, die als Ausgangsstoffe für die Proteinhydrolysate dienen, können sowohl pflanzlicher als auch tierischer Herkunft sein. Übliche Ausgangsstoffe sind z.B. Keratin, Collagen, Elastin, Sojaprotein, Reisprotein, Milchprotein, Weizenprotein, Seidenprotein oder Mandelprotein. Durch die Hydrolyse entstehen Stoffmischungen mit Molmassen im Bereich von ca. 100 bis ca. 50.000. Übliche mittlere Molmassen liegen im Bereich von etwa 500 bis etwa 1000. Vorteilhafterweise enthalten die kationisch derivatisierten Proteinhydrolysate eine oder zwei lange C8- bis C22-Alkylketten und entsprechend zwei oder eine kurze C1- bis C4-Alkylketten. Verbindungen, die eine lange Alkylkette enthalten, sind bevorzugt.

Als Haarpflegewirkstoff (B) besonders bevorzugt sind kationaktive Silikonverbindungen. Diese sind mit kationischen oder kationisierbaren Gruppen substituiert. Geeignete kationaktive Silikonvberbindungen weisen entweder mindestens eine Aminogruppe oder mindestens eine Ammoniumgruppe auf. Silikonpolymere mit Aminogruppen sind unter den INCI-Bezeichnungen Amodimethicone und Trimethylsilylamodimethicone bekannt. Hierbei handelt es sich um Polydimethylsiloxane mit Aminoalkylgruppen. Die Aminoalkylgruppen können seiten- oder endständig sein. Geeignete Aminosilikone sind solche der allgemeinen Formel (VI)

R⁸R⁹R¹⁰Si- (OSiR¹¹R¹²)x- (OSiR¹³Q)y-OSiR¹⁴R¹⁵R¹⁶ (VI)

R⁸, R⁹, R¹⁴ und R¹⁵ sind unabhängig voneinander gleich oder verschieden und bedeuten C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl oder Trimethylsilyl; R¹⁰ und R¹⁶ sind unabhängig voneinander gleich oder verschieden und bedeuten -(CH₂)ₐ-NH₂ mit a gleich 1 bis 6, C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;
R¹¹, R¹² und R¹³ sind unabhängig voneinander gleich oder verschieden und bedeuten Wasserstoff, C1- bis C20-Kohlenwasserstoff, welcher O- und N-Atome enthalten kann, vorzugsweise C1- bis C10-Alkyl oder Phenyl, besonders bevorzugt C1-bis C4-Alkyl, insbesondere Methyl;
Q bedeutet -A-NR¹⁷R¹⁸, oder -A-N⁺R¹⁷R¹⁸R¹⁹ wobei A für eine divalente C1- bis C20-Alkylenverbindungsgruppe steht, welche auch O- und N-Atome sowie OH-Gruppen enthalten kann, und
R¹⁷, R¹⁸ und R¹⁹ unabhängig voneinander gleich oder verschieden sind und Wasserstoff, C1- bis C22-Kohlenwasserstoff, vorzugsweise C1- bis C-4-Alkyl oder Phenyl bedeuten. Bevorzugte Reste für Q sind -(CH₂)₃-NH₂, (CH₂)₃NHCH₂CH₂NH₂, - (CH₂)₃OCH₂CHOHCH₂NH₂ und - (CH₂)₃N(CH₂CH₂OH)₂, -(CH₂)₃-NH₃⁺ und -(CH₂)₃OCH₂CHOHCH₂N⁺(CH₃)₂R²⁰, wobei R²⁰ ein C1- bis C22-Alkylrest ist, der auch OH-Gruppen aufweisen kann; x bedeutet eine Zahl zwischen 1 und 10.000, vorzugsweise zwischen 1 und 1.000; y bedeutet eine Zahl zwischen 1 und 500, vorzugsweise zwischen 1 und 50. Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 0,1 bis 0,5.

Geeignete Silikonpolymere mit zwei endständigen quaternären Ammoniumgruppen sind unter der INCI-Bezeichnung Quaternium-80 bekannt. Hierbei handelt es sich um Dimethylpolysiloxane mit 2 endständigen Alkylammoniumgruppen. Geeignete quaternäre Aminosilikone sind solche der allgemeinen Formel (VII)

R²¹R²²R²³N⁺-A-SiR⁸R⁹- (OSiR¹¹R¹²)ₙ-OSiR⁸R⁹-A-N⁺R²¹R²²R²³ 2X⁻ (VII)

A hat die gleiche Bedeutung wie oben bei Formel (VI) angegeben und ist vorzugsweise (CH₂)₃OCH₂CHOHCH₂N⁺(CH₃)₂R²⁰
wobei R²⁰ ein C1- bis C22-Alkylrest ist, der auch OH-Gruppen aufweisen kann;
R⁸, R⁹, R¹¹ und R¹² haben die gleiche Bedeutung wie oben bei Formel (VI) angegeben und sind vorzugsweise Methyl;
R²¹, R²², und R²³ bedeuten unabhängig voneinander C1-bis C22-Alkylreste, welche Hydroxygruppen enthalten können und wobei vorzugsweise mindestens einer der Reste mindestens 10 C-Atome aufweist und die übrigen Reste 1 bis 4 C-Atome aufweisen; n ist eine Zahl von 0 bis 200, vorzugsweise 10 bis 100. Derartige diquaternäre Polydimethylsiloxane werden von der Firma GOLDSCHMIDT/Deutschland unter den Handelsnamen Abil® Quat 3270, 3272 und 3274 vertrieben.

Die Perlglanz- oder Trübungsmittel (C) sind ausgewählt aus Fettsäurealkanolamiden, Fettsäureglycerylestern, Guanin, Glykoldifettsäureestern, Styrol/Acrylat Copolymeren, Polyethylenglykoldifettsäureestern, Styrol/Vinylpyrrolidon Copolymeren und Poly(trimethylammoniumethylmethacrylat Chlorid). Geeignete Fettsäurealkanolamide sind solche der allgemeinen Formel (VIII)

R¹-C(=O) -NR²R³ (VIII)

wobei R1 einen gesättigten oder einfach oder mehrfach ungesättigten C7- bis C21-Kohlenwasserstoffrest bedeutet, R2 einen C1- bis C4-Alkylrest bedeutet, der mit mindestens einer OH-Gruppe substituiert ist und R3 Wasserstoff oder einen C1- bis C4-Alkylrest bedeutet, der mit mindestens einer OH-Gruppe substituiert ist. Vorzugsweise steht R2 für CH2CH2OH, R3 für Wasserstoff oder CH2CH2OH und R1-C(=O) für einen Fettsäurerest. Besonders bevorzugt sind Kokosfettsäuremono- und -diethanolamid (INCI: Cocamide MEA, Cocamide DEA). Geeignete Fettsäureglycerylester sind solche der allgemeinen Formel R-C(=O)-OCH₂CH(OH)CH₂OH, wobei R für einen gesättigten oder einfach oder mehrfach ungesättigten C7- bis C21-Kohlenwasserstoffrest steht. Vorzugsweise ist R-C(=O) ein Fettsäurerest, besonders bevorzugt ist Glycerylmonolaurat. Geeignete Glykoldifettsäureester sind solche der allgemeinen Formel RC(=O)O-(CH₂CH₂O)ₙ-C(=O)R, wobei n gleich 1 ist und R für einen gesättigten oder einfach oder mehrfach ungesättigten C7- bis C21-Kohlenwasserstoffrest steht. Vorzugsweise ist R-C(=O) ein Fettsäurerest, besonders bevorzugt ist Ethylenglykoldistearat. Geeignete Polyethylenglykoldifettsäureester sind solche der vorgenannten Formel, wobei n für eine Zahl von 2 bis 6 steht. Besonders bevorzugt ist PEG-3 Distearat. Geeignete Styrol/Acrylat Copolymere sind Copolymere aus Styrol und mindestens einem Monomer, ausgewählt aus Acrylsäure, Methacrylsäure oder deren C1-C4-Alkylestern, Methacrylamid und Acrylamid (INCI: Styrene/Acrylates Copolymer, Styrene/Acrylamide Copolymer). Ein geeignetes Poly(trimethylammoniumethylmethacrylat Chlorid) (INCI: Polyquaternium-37) ist im Handel unter der Bezeichnung Salcare® SC96 (Ciba, Allied Colloids) erhältlich.

Ein besonders schöner und besonders stabiler Perlglanzeffekt wird erreicht mit einer Mischung aus mindestens einem Fettsäurealkanolamid und mindestens einem Glykoldifettsäureester, insbesondere einer Mischung aus Kokosfettsäuremonooder -diethanolamid und Glykoldistearat. Diese Mischung wird dabei vorzugsweise in einer Menge von 0,1 bis 10, besonders bevorzugt von 1 bis 5 Gew.% und in einem Mengenverhältnis von Fettsäurealkanolamid zu Glykoldifettsäureester von 0,8:1 bis 3:1 eingesetzt.

Das erfindungsgemäße Mittel wird in einem wäßrigen Milieu konfektioniert. Der Wasseranteil beträgt dabei in der Regel 70 bis 98, besonders bevorzugt 80 bis 95 Gew.%. Als Nebenbestandteile können übliche Co-Solventien enthalten sein, z.B. die für kosmetische Zwecke üblicherweise verwendeten niederen Monoalkohole mit 2 bis 5 C-Atomen wie z.B. Ethanol und Isopropanol oder mehrwertige Alkohole, insbesondere solche mit 2 bis 5 C-Atomen wie z.B. Glycerin, Ethylenglykol, Propylenglykol, Butylenglykol oder Pentandiol. Der Alkoholgehalt beträgt vorzugsweise von 0,1 bis 10, besonders bevorzugt von 0,5 bis 5 Gew.%.

In einer bevorzugten Ausführungsform enthält das erfindungsgemäße Mittel zusätzlich mindestens ein nichtionisches Tensid. Geeignete nichtionische Tenside sind beispielsweise die im "International Cosmetic Ingredient Dictionary and Handbook", 7. Auflage, Band 2 im Abschnitt "Surfactants - Emulsifying Agents" aufgeführten nichtionischen Emulgatoren. Geeignete nichtionische Tenside sind vorzugsweise ausgewählt aus ethoxylierten Fettsäuren mit 10 bis 26 C-Atomen, ethoxylierten ein- oder mehrwertigen Alkoholen mit 1 bis 6 C-Atomen, ethoxylierten Fettalkoholen mit 10 bis 26 C-Atomen, ethoxyliertem hydriertem oder nicht hydriertem Rizinusöl, Alkylpolyglucosiden, Glyceridalkoxylaten, Fettsäureglyceridpolyalkylenglykolethern oder Fettsäurepartialglyceridpolyalkylenglykolethern mit jeweils weniger als 30 Alkylenglykoleinheiten wie z.B. Polyethylenglykol-(7)-glycerylcocoat, Polyglykolamiden, Fettsäurezuckerestern, ethoxylierten Fettsäurezuckerestern und Partialglyceriden. Der Ethoxylierungsgrad von ethoxylierten Tensiden beträgt üblicherweise von 1 - 400, vorzugsweise 2 - 200, besonders bevorzugt 3 - 25. Bevorzugte nichtionische Tenside sind insbesondere Fettalkoholethoxylate. Geeignet sind z.B. Alkohole mit 10 bis 18, vorzugsweise 10 bis 16 C-Atomen und einem Ethoxylierungsgrad von vorzugsweise 2 bis 200, besonders bevorzugt von 3 bis 25. Die zusätzlichen nichtionischen Tenside werden in einer Menge von vorzugsweise 0,01 bis 5 Gew.% eingesetzt.

Das erfindungsgemäße Mittel kann darüber hinaus die für Haarbehandlungsmittel üblichen Zusatzbestandteile enthalten, z.B. nichtionische oder anionische Polymere, soweit sie mit den übrigen Bestandteilen verträglich sind, in einer Menge von vorzugsweise 0,01 bis 10 Gew. %; Parfümöle in einer Menge von vorzugsweise 0,01 bis 5 Gew. %; Netzmittel oder Emulgatoren in einer Menge von vorzugsweise 0.01 bis 10 Gew. %; Feuchthaltemittel; Konservierungsmittel, bakterizide und fungizide Wirkstoffe wie z.B. 2,4,4-Trichlor-2-hydroxydiphenylether, Parabene oder Methylchlorisothiazolinon, in einer Menge von 0,01 bis 1,0 Gew.%; Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Pflanzen- und Kräuterextrakte, Protein- und Seidenhydrolysate, Lanolinderivate, in einer Menge von 0,1 bis 5 Gew.%; Lichtschutzmittel, Antioxidantien, Radikalfänger, Antischuppenwirkstoffe, Glanzgeber, Vitamine und rückfettende Agenzien in einer Menge von 0,01 bis 10 Gew.%.

Das erfindungsgemäße Mittel kann in einem pH-Bereich von 2,0 bis 9,5 vorliegen. Besonders bevorzugt sind schwach saure pH-Werte im Bereich zwischen 4,5 und kleiner 7, besonders bevorzugt bis 6,5. Liegt das erfindungsgemäße Mittel im sauren Bereich vor, so kann es organische oder anorganische Säuren enthalten wie z.B. Ameisensäure, Weinsäure, Äpfelsäure, Maleinsäure, Fumarsäure, Glyoxylsäure, Pyrrolidoncarbonsäure, Zitronensäure, Milchsäure, Schwefelsäure, Essigsäure, Salzsäure, Phosphorsäure u.a..

Das erfindungsgemäße Mittel liegt vorzugsweise in Form einer viskosen Creme oder einer fließfähigen Emulsion vor. Die Viskosität liegt dabei vorzugsweise im Bereich von 300 bis 6000 mPa s, vorzugsweise von 500 bis 4000 mPa s, gemessen als dynamische Viskositätsmessung mit einem HAAKE VT-550 Rheometer, Messkörper SV-DIN bei einer Temperatur von 25°C und einer Schergeschwindigkeit von 50 s⁻¹. Die Abfüllung erfolgt je nach Konsistenz in eine geeignete Verpackung, z.B. in Tuben, Cremetiegeln, Glas- oder Kunststoffflaschen, Pumpspender oder zusammen mit eidnem Treibgas in einem Aerosolbehälter. Hierbei ist das Verpackungsmaterial vorzugsweise transparent, durchsichtig oder zumindest durchscheinend, z.B. aus Glas oder durchsichtigem Kunststoff, z.B. Polyethylen, Polypropylen oder Polyethylenterephtalat.

Zur Herstellung der erfindungsgemäßen Mittel werden die Perlglanz- oder Trübungsmittel, sofern sie in fester Form vorliegen, aufgeschmolzen und bei erhöhter Temperatur (z.B. 50-90 °C, vorzugsweise 60-85°C) in eine wässrige Lösung der übrigen Bestandteile mit Ausnahme des Verdickers einemulgiert. Die erkaltete Masse wird dann zu einer wässrigen Lösung des Assoziativverdickers gegeben. Flüssige Perlglanzkonzentrate lassen sich direkt zur Masse kalt dazurühren.

Das Mittel wird angewendet, indem eine für den gewünschten Konditioniereffekt ausreichende Menge in oder auf dem trockenen Haar oder nach der Haarwäsche in oder auf dem nassen oder feuchten Haar verteilt wird. Die anzuwendende Menge hängt von der Haarfülle ab und beträgt typischerweise 1 bis 25 g. Bei der bevorzugten Verwendung als Rinse-Produkt wird nach einer ausreichenden Einwirkzeit von beispielsweise 1 bis 15 Minuten das Haar ausgespült. Anschließend wird das Haar gegebenenfalls durchgekämmt oder zur Frisur geformt und getrocknet. Bei einer Verwendung als Leave-in-Produkt wird das Haar nach Aufbringen des Mittels nicht ausgespült.

Die Mittel zeichnen sich dadurch aus, dass sie mit reproduzierbarer Qualität herstellbar sind, weil Produktkonsistenz und Perlglanz weniger stark von einem schwankenden Abkühlverhalten beeinflußt werden. Die Produkte zeichnen sich außerdem durch eine erhöhte Stabilität aus. Auch nach 3 Monate Lagerung bei 40 °C zeigten sich keine Veränderungen von Viskosität und Perlglanz.

Die nachfolgenden Beispiele sollen den Gegenstand der Erfindung näher erläutern.

### Beispiel 1: Perlglänzende Haarkur

| | |
|---|---|
| 3,0 g | Kokosfettsäuremonoethanolamid |
| 2,0 g | Abil® Quat 3272 (Quaternium-80, 50%ig in Propylenglykol) |
| 1,8 g | Propylenglykol |
| 1,2 g | Polyether-1 (Pure Thix® TX-1442) |
| 0,75 | Cetyltrimethylammoniumchlorid |
| 0,7 g | Tegobetain® (30%ig in Wasser, Cocamidopropyl Betaine) |
| 0,5 g | Laureth-4 |
| ad 100 g | Wasser |

### Beispiel 2: Perlglänzende Haarkur

| | |
|---|---|
| 3,33 g | Euperlan® PK 4000 (40% Glykoldistearat, 7,5% Laureth-4, 7,5% Cocamidopropylbetain in Wasser) |
| 2,0 g | Kokosfettsäuremonoethanolamid |
| 2,0 g | Abil® Quat 3272 (Quaternium-80, 50%ig in Propylenglykol) |
| 1,8 g | Propylenglykol |
| 1,2 g | Polyether-1 (Pure Thix® TX-1442) |
| 0,75 | Cetyltrimethylammoniumchlorid |
| 0,7 g | Tegobetain® (30%ig in Wasser, Cocamidopropyl Betaine) |
| 0,5 g | Laureth-4 |
| ad 100 g | Wasser . |

Viskosität: ca. 2000 mPa s, gemessen als dynamische Viskositätsmessung mit einem HAAKE VT-550 Rheometer, Messkörper SV-DIN bei einer Temperatur von 25°C und einer Schergeschwindigkeit von 50 s⁻¹.
Die Haarkur wies auch bei 3 Monaten Lagerung bei 40°C gleichbleibende Viskosität und einen stabilen Perlglanzeffekt auf.

## Patentansprüche

1. Perlglänzendes Haarpflegemittel mit einem Gehalt an
(A) mindestens einem nichtionischen, amphiphilen Assoziativverdicker, ausgewählt aus hydrophob modifizierten Aminoplast/Polyether Copolymeren,
(B) mindestens einem Haarpflegewirkstoff, ausgewählt aus kationischen Tensiden, zwitterionischen Tensiden, kationischen Polymeren, kationischen Silikonverbindungen, aminsubstituierten Silikonverbindungen, kationisch derivatisierten Proteinen, kationisch derivatisierten Proteinhydrolysaten und Betain und
(C) mindestens einem Perlglanz- oder Trübungsmittel, ausgewählt aus Fettsäurealkanolamiden, Fettsäureglycerylestern, Guanin, Glykoldifettsäureestern, Styrol/Acrylat Copolymeren, Polyethylenglykoldifettsäureestern, Styrol/Vinylpyrrolidon Copolymeren und Poly(trimethylammoniumethylmethacrylat Chlorid)
in einer wässrigen, kosmetischen Basis.

2. Mittel nach Anspruch 1, **dadurch gekennzeichnet, dass** der Assoziativverdicker (A) in einer Menge von 0,1 bis 5 Gew.%; der Haarpflegewirkstoff (B) in einer Menge von 0,01 bis 10 Gew.% und das Perlglanz- oder Trübungsmittel (C) in einer Menge von 0,1 bis 10 Gew.% enthalten sind.

3. Mittel nach einem der vorhergehenden Ansprüche, **dadurch gekennzeichnet, dass** der Assoziatiwerdicker (A) ausgewählt ist aus Polymeren der allgemeinen Formel 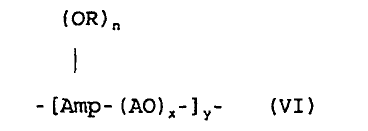 wobei Amp ein Aminoplastmonomer oder den Rest eines Aminoplastoligomers oder -polymers bedeutet, AO für eine Alkylenoxidgruppe steht, R für Wasserstoff, C1-C4-Alkyl oder C1-C4-Acyl steht, x und y Zahlen größer 1 sind und n eine positive Zahl ist.

4. Mittel nach einem der vorhergehenden Ansprüchen, **dadurch gekennzeichnet, dass** der Assoziativverdicker (A) ausgewählt ist aus den Reaktionsprodukten der säurekatalysierten Reaktion von Glycolurilderivaten und Polyalkylenglykolen und alkoxylierten Kohlenwasserstoffen.

5. Mittel nach einem der vorhergehenden Ansprüchen, **dadurch gekennzeichnet, dass** der Assoziativverdicker (A) ausgewählt ist aus Polyether-1, PEG-180/Octoxynol-40/TMMG Copolymer und PEG-180/Laureth-50/TMMG Copolymer.

6. Mittel nach einem der vorhergehenden Ansprüche, **dadurch gekennzeichnet, dass** der Haarpflegewirkstoff (B) ausgewählt ist aus
- kationischen Tensiden der allgemeinen Formel (I)
N ⁽⁺⁾R¹R²R³R⁴ X⁽⁻⁾ (I)
wobei R1 bis R4 unabhängig voneinander aliphatische Gruppen, aromatische Gruppen, Alkoxygruppen, Polyoxyalkylengruppen, Alkylamidogruppen, Hydroxyalkylgruppen oder Alkarylgruppen mit 1 bis 22 C-Atomen bedeuten, wobei mindestens einer der Substituenten mindestens 8 C-Atome aufweist und X⁽⁻⁾ ein kosmetisch verträgliches Anion darstellt,
- kationischen organischen Polymeren, ausgewählt aus Methylvinylimidazoliumchlorid/Vinylpyrrolidon Copolymeren, quaternisierten Vinylpyrrolidon/Dimethylaminoethylmethacrylat Copolymeren, kationisch derivatisierten Polysacchariden, neutralisiertem Chitosan und neutralisierten Chitosanderivaten
- kationischen Silikonpolymeren, ausgewählt aus Dimethylpolysiloxanen mit endständigen Alkylammoniumgruppen.

7. Mittel nach einem der vorhergehenden Ansprüche, **dadurch gekennzeichnet, dass** das Perlglanz- oder Trübungsmittel (C) ausgewählt ist aus Fettsäuremono- oder -dialkanolamiden der allgemeinen Formel R¹-C(=O)-NR²R³ wobei R¹ für eine Alkyl- oder für eine ein- oder mehrfach ungesättigte Alkenylgruppe mit 7 bis 21 C-Atomen steht, R² für eine Alkylgruppe mit 1 bis 4 C-Atomen steht, welche mit mindestens einer OH-Gruppe substituiert ist und R3 für Wasserstoff oder für eine Alkylgruppe mit 1 bis 4 C-Atomen steht, welche mit mindestens einer OH-Gruppe substituiert ist.

8. Mittel nach einem der vorhergehenden Ansprüche, **dadurch gekennzeichnet, dass** als Perlglanz- oder Trübungsmittel (C) 1 bis 5 Gew.% einer Kombination von mindestens einem Fettsäuremono- oder -dialkanolamid und mindestens einem Glykoldifettsäureester im Verhältnis von 0,8:1 bis 3:1 enthalten ist.

9. Mittel nach einem der vorhergehenden Ansprüche, **dadurch gekennzeichnet, dass** es eine Viskosität von 300 bis 6000 mPa s aufweist bei 25°C und einer Schergeschwindigkeit von 50 s⁻¹.

10. Mittel nach einem der vorhergehenden Ansprüche, **gekennzeichnet durch** einen Gehalt an
(A) 0,1 bis 5 Gew.% eines Assoziativverdickers, ausgewählt aus Polyether-1, PEG-180/Octoxynol-40/TMMG Copolymer und PEG-180/Laureth-50/TMMG Copolymer,
(B) 0,01 bis 10 Gew.% eines Haarpflegewirkstoffs, ausgewählt aus kationischen Tensiden und kationischen Silikonen und
(C) 0,1 bis 10 Gew.% Fettsäuremono- oder -diethanolamid oder eines Gemisches aus einem Fettsäuremono- oder - diethanolamid und Glykoldifettsäureester.

11. Haarpflegeprodukt bestehend aus einer durchsichtigen oder durchscheinenden Verpackung und einer Zusammensetzung gemäß einer der Ansprüche 1 bis 10.

## Claims

1. Pearlescent haircare composition with a content of
(A) at least one non-ionic, amphiphilic associative thickener chosen from hydrophobically modified aminoplast/polyether copolymers,
(B) at least one haircare active ingredient chosen from cationic surfactants, zwitterionic surfactants, cationic polymers, cationic silicone compounds, amine-substituted silicone compounds, cationically derivatized proteins, cationically derivatized protein hydrolysates and betaine and
(C) at least one pearlescent agent or opacifier chosen from fatty acid alkanolamides, fatty acid glyceryl esters, guanine, glycol difatty acid esters, styrene/acrylate copolymers, polyethylene glycol difatty acid esters, styrene/vinylpyrrolidone copolymers and poly(trimethylammonium ethyl methacrylate chloride)
in an aqueous, cosmetic base.

2. Composition according to Claim 1, **characterized in that** the associative thickener (A) is present in an amount of from 0.1 to 5% by weight; the haircare active ingredient (B) is present in an amount of from 0.01 to 10% by weight, and the pearlescent agent or opacifier (C) is present in an amount of from 0.1 to 10% by weight.

3. Composition according to one of the preceding claims, **characterized in that** the associative thickener (A) is chosen from polymers of the general formula 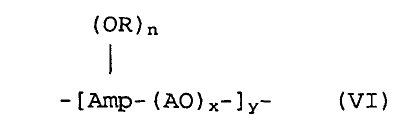 where Amp is an aminoplast monomer or the radical of an aminoplast oligomer or polymer, AO is an alkylene oxide group, R is hydrogen, Cl-C4-alkyl or C1-C4-acyl, x and y are numbers greater than 1 and n is a positive number.

4. Composition according to one of the preceding claims, **characterized in that** the associative thickener (A) is chosen from the reaction products of the acid-catalysed reaction of glycoluril derivatives and polyalkylene glycols and alkoxylated hydrocarbons.

5. Composition according to one of the preceding claims, **characterized in that** the associative thickener (A) is chosen from polyether-1, PEG-180/octoxynol-40/TMMG copolymer and PEG-180/laureth-50/TMMG copolymer.

6. Composition according to one of the preceding claims, **characterized in that** the haircare active ingredient (B) is chosen from
- cationic surfactants of the general formula (I)
N⁽⁺⁾R¹R²R³R⁴ X⁽⁻⁾ (I)
where R¹ to R⁴, independently of one another, are aliphatic groups, aromatic groups, alkoxy groups, polyoxyalkylene groups, alkylamido groups, hydroxyalkyl groups or alkaryl groups having 1 to 22 carbon atoms, where at least one of the substituents has at least 8 carbon atoms and X⁽⁻⁾ is a cosmetically compatible anion,
- cationic organic polymers chosen from methylvinylimidazolium chloride/vinyl-pyrrolidone copolymers, quaternized vinylpyrrolidone/dimethylaminoethyl methacrylate copolymers, cationically derivatized polysaccharides, neutralized chitosan and neutralized chitosan derivatives
- cationic silicone polymers chosen from dimethylpolysiloxanes with terminal alkylammonium groups.

7. Composition according to one of the preceding claims, **characterized in that** the pearlescent agent or opacifier (C) is chosen from fatty acid mono- or dialkanolamides of the general formula R¹-C(=O)-NR²R³, where R¹ is an alkyl group or a mono- or polyunsaturated alkenyl group having 7 to 21 carbon atoms, R² is an alkyl group having 1 to 4 carbon atoms which is substituted by at least one OH group, and R³ is hydrogen or an alkyl group having 1 to 4 carbon atoms which is substituted by at least one OH group.

8. Composition according to one of the preceding claims, **characterized in that** the pearlescent agent or opacifier (C) present is 1 to 5% by weight of a combination of at least one fatty acid mono- or dialkanolamide and at least one glycol difatty acid ester in the ratio from 0.8:1 to 3:1.

9. Composition according to one of the preceding claims, **characterized in that** it has a viscosity from 300 to 6000 mPa s at 25°C and shear rate of 50 s⁻¹.

10. Composition according to one of the preceding claims, **characterized by** a content of
(A) 0.1 to 5% by weight of an associative thickener chosen from polyether-1, PEG-180/octoxynol-40/TMMG copolymer and PEG-180/laureth-50/TMMG copolymer,
(B) 0.01 to 10% by weight of a haircare active ingredient chosen from cationic surfactants and cationic silicones and
(C) 0.1 to 10% by weight of fatty acid mono- or diethanolamide or of a mixture of a fatty acid mono- or diethanolamide and glycol difatty acid ester.

11. Haircare product consisting of a transparent or translucent packaging and a composition according to one of Claims 1 to 10.

## Revendications

1. Composition de soin capillaire nacrée ayant une teneur en
(A) au moins un épaississant associatif amphiphile non ionique choisi parmi des copolymères aminoplaste/polyéther à modification hydrophobe,
(B) au moins une substance active de soin capillaire choisie parmi des tensioactifs cationiques, des tensioactifs zwitterioniques, des polymères cationiques, des silicones cationiques, des silicones substitués par des groupes amino, des protéines à fonctionnalisation cationique, des hydrolysats de protéines à fonctionnalisation cationique et la bétaïne et
(C) au moins un agent nacrant ou opacifiant choisi parmi des alcanolamides d'acides gras, des esters glycéryliques d'acides gras, la guanine, des diesters d'acides gras et glycol, des copolymères styrène/acrylate, des diesters d'acides gras et polyéthylèneglycol, des copolymères styrène/vinylpyrrolidone et le poly(chlorureméthylméthacrylate de triméthylammonium)
dans une base cosmétique aqueuse.

2. Composition selon la revendication 1, **caractérisée en ce que** l'épaississant associatif (A) est contenu en une quantité de 0,1 à 5 % en poids ; la substance active de soin capillaire (B) est contenue en une quantité de 0,01 à 10 % en poids et l' agent nacrant ou opacifiant (C) est contenu en une quantité de 0,1 à 10 % en poids.

3. Composition selon l'une quelconque des revendications précédentes, **caractérisée en ce que** l'épaississant associatif (A) est choisi parmi des polymères de formule générale 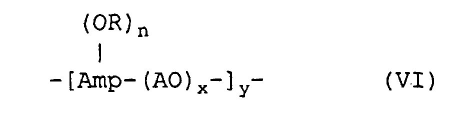 dans laquelle Amp représente un monomère aminoplaste ou le reste d'un, oligomère ou polymère aminoplaste, AO représente un groupe oxyde d'alkylène, R représente un atome d'hydrogène, un groupe alkyle en C₁-C₄ ou acyle en C₁-C₄, x et y sont des nombres supérieurs à 1 et n est un nombre positif.

4. Composition selon l'une quelconque des revendications précédentes, **caractérisée en ce que** l'épaississant associatif (A) est choisi parmi les produits de réaction de la réaction, catalysée par un acide, de dérivés de glycolurile et de polyalkylèneglycols et d'hydrocarbures alcoxylés.

5. Composition selon l'une quelconque des revendications précédentes, **caractérisée en ce que** l'épaississant associatif (A) est choisi parmi le polyéther-1, le copolymère PEG-180/octoxynol-40/TMMG et le copolymère PEG-180/laureth-50/TMMG.

6. Composition selon l'une quelconque des revendications précédentes, **caractérisée en ce que** la substance de soin capillaire (B) est choisie parmi
- des tensioactifs cationiques de formule générale (I)
N ⁽⁺⁾ R¹R²R³R⁴ X⁽⁻⁾ (I)
dans laquelle R¹ à R⁴ représentent, indépendamment les uns des autres, des groupes aliphatiques, des groupes aromatiques, des groupes alcoxy, des groupes polyoxyalkylène, des groupes alkylamido, des groupes hydroxyalkyle ou des groupes alkaryle ayant de 1 à 22 atomes de carbone, au moins l'un des substituants ayant au moins 8 atomes de carbone et X⁽⁻⁾ représentant un anion cosmétiquement acceptable,
- des polymères organiques cationiques choisis parmi des copolymères chlorure de méthylvinylimidazolium/vinylpyrrolidone, des copolymères vinylpyrrolidone/méthacrylate de diméthylaminoéthyle rendus quaternaires, des polysaccharides à fonctionnalité cationique, le chitosane neutralisé et des dérivés de chitosane neutralisés,
- des polymères silicone cationiques choisis parmi des diméthylpolsiloxanes à groupes alkylammonium en bout de chaîne.

7. Composition selon l'une quelconque des revendications précédentes, **caractérisée en ce que** l'agent nacrant ou opacifiant (C) est choisi parmi des mono- ou dialcanolamides d'acides gras de formule générale R¹-C(= O)-NR²R³, dans laquelle R¹ représente un groupe alkyle ou un groupe alcényle une ou plusieurs fois insaturé ayant de 7 à 21 atomes de carbone, R² représente un groupe alkyle ayant de 1 à 4 atomes de carbone, qui est substitué par au moins un groupe OH et R³ représente un atome d'hydrogène ou un groupe alkyle ayant de 1 à 4 atomes de carbone, qui est substitué par au moins un groupe OH.

8. Composition selon l'une quelconque des revendications précédentes, **caractérisée en ce qu'**elle contient en tant qu'agent nacrant ou opacifiant (C) 1 à 5 % en poids d'une association d'au moins un mono- ou dialcanolamide d'acide gras et d'au moins un diester d'acide gras et glycol en un rapport de 0,8:1 à 3:1.

9. Composition selon l'une quelconque des revendications précédentes, **caractérisée en ce qu'**elle présente une viscosité de 300 à 6 000 mPa.s à 25°C et une vitesse de cisaillement de 50 s⁻¹.

10. Composition selon l'une quelconque des revendications précédentes, **caractérisée par** une teneur de
(A) 0,1 à 5 % en poids d'un épaississant associatif choisi parmi le polyéther-1, le copolymère PEG-180/octoxynol-40/TMMG et le copolymère PEG-180/laureth-50/TMMG,
(B) 0,01 à 10 % en poids d'une substance active de soin capillaire choisie parmi des tensioactifs cationiques et des silicones cationiques et
(C) 0,1 à 10 % en poids de mono- ou diéthanolamide d'acide gras ou d'un mélange de mono- ou diéthanolamide d'acide gras et de diester d'acide gras et glycol.

11. Produit de soin capillaire constitué d'un conditionnement transparent ou translucide et d'une composition selon l'une quelconque des revendications 1 à 10.
